# EUROPÄISCHE PATENTSCHRIFT

(11) **EP 1 966 188 B1**
(45) Veröffentlichungstag und Bekanntmachung des Hinweises auf die Patenterteilung: **03.11.2010**
(21) Anmeldenummer: 06742610.6
(22) Anmeldetag: 19.04.2006
(51) Int. Cl.: C07D 401/12, C07D 235/28

(54) **ENANTIOSELEKTIVE HERSTELLUNG VON BENZIMIDAZOLDERIVATEN UND IHREN SALZEN**
ENANTIOSELECTIVE PREPARATION OF BENZIMIDAZOLE DERIVATIVES AND THEIR SALTS
FABRICATION ENANTIOSELECTIVE DE DERIVES DE BENZIMIDAZOLE ET DE LEURS SELS

(30) Priorität: 22.12.2005 DE 102005061720
(43) Veröffentlichungstag der Anmeldung: 10.09.2008
(73) Patentinhaber: Ratiopharm GmbH, 89079 Ulm (DE)
(72) Erfinder: JIANG, Biao, Shanghai 200032 (CN); ZHAO, Xiao-Long, Shanghai 200032 (CN); DONG, Jia-Jia, Shanghai 200032 (CN); WANG, Wan-Jun SIOC-Shanghai Institute of Organic Chemistry, Shanghai 200032 (CN)
(74) Vertreter: Best, Michael
(86) Internationale Anmeldenummer: PCT/EP2006/003587
(87) Internationale Veröffentlichungsnummer: WO 2007/079784

(56) Entgegenhaltungen:
- EP-A- 1 277 752
- WO-A-03/089408
- WO-A-2004/002982
- WO-A-2004/099182
- US-A- 5 948 789

## Beschreibung

Die Erfindung betrifft ein neues Verfahren zur Herstellung von Benzimidazolderivaten mit einer chiralen Sulfoxidgruppen in enantiomerenreiner Form oder in einer Form, in der eines der beiden Enantiomeren gegenüber dem anderen Enantiomeren angereichert ist. Die Erfindung betrifft ebenfalls ein Verfahren zur Herstellung der Salze der einzelnen Enantiomere der Benzimidazolderivate mit einer chiralen Sulfoxidgruppe. Insbesondere betrifft die Erfindung ein Verfahren zur Herstellung des S-Enantiomers des Omeprazols (auch als Esomeprazol bekannt) und der Salze davon, insbesondere des Zinksalzes des S-Enantiomers des- Omeprazols. Die Begriffe S-Enantiomer des Omeprazols und Esomeprazol werden in dieser Anmeldung synonym verwendet.

Benzimidazolderivate mit einer chiralen Sulfoxidgruppe sind bekannte Inhibitoren der Magensäuresekretion, und viele Verbindungen aus dieser Gruppe werden als Arzneimittel zur Behandlung von Magen-Darmerkrankungen, insbesondere von Magengeschwüren, eingesetzt. Hier können z.B. die bekannten Wirkstoffe Omeprazol, Pantoprazol, Lansoprazol und Rabeprazol genannt werden. Die Wirkstoffe sind aufgrund der Sulfoxidgruppe chiral, so dass ein Interesse daran besteht, die Verbindungen in enantiomerenreiner Form herzustellen. Insbesondere das S-Enantiomer des Omeprazols, das Esomeprazol, wird derzeit in Form seines Magnesiumsalzes umfangreich vermarktet.

Die Auftrennung von substituierten 2-(2-Pyridinylmethylsulfinyl)-1H-benzimidazolen in die einzelnen Enantiomere ist beispielsweise in der DE 40 35 455, der WO 94/27988 und der WO 2004/002982 beschrieben. Diese Druckschriften betreffen insbesondere auch die Auftrennung des Omeprazols in seine beiden Enantiomere. Die in diesen Druckschriften beschriebenen Verfahren gehen von dem Racemat der Verbindungen aus und überführen das Racemat durch Umsetzung mit einer optisch aktiven Verbindung in ein Diastereomerenpaar, das dann auf übliche Art und Weise aufgetrennt werden kann. Das Isolieren eines Enantiomers aus einem an diesem Enantiomer angereicherten Gemisch zweier Enantiomere chiraler Benzimidazolverbindungen ist auch in der WO 97/02261 beschrieben. Derartige Verfahren zur Auftrennung eines racemischen Gemischs haben eine Reihe von Nachteilen, da zum einen das nicht gewünschte Enantiomer in der Regel verworfen werden muss und zum anderen die Auftrennung mit komplexen und die Ausbeute vermindernden Schritten verbunden ist.

Dementsprechend.gibt es auf dem Gebiet eine Reihe von Vorschlägen, die einzelnen Enantiomere der Benzimidazolderivate mit einer chiralen Sulfoxidgruppe durch eine chirale Synthese herzustellen.

Hier kann beispielsweise auf die WO 96/17076 verwiesen werden, die die mikrobielle Oxidation der entsprechenden prochiralen Sulfide zu den einzelnen Enantiomeren der gewünschten Sulfoxidverbindungen offenbart, oder die WO 96/17077, die die mikrobielle Reduktion von racemischen Sulfonen zu den gewünschten Stereoisomeren der Sulfoxide beschreibt. Bei diesen Verfahren handelt es sich aber um mikrobielle Verfahren, und es wäre wünschenswert, ein chemisches Verfahren zur asymmetrischen Synthese der entsprechenden einzelnen Enantiomere der Benzimidazolderivate mit einer chiralen Sulfoxidgruppe zu erhalten.

Die WO 96/002535 offenbart ein derartiges Verfahren, bei dem ein prochirales Sulfid mit einem Oxidationsmittel in Gegenwart eines Katalysators umgesetzt wird. Bei dem Katalysator handelt es sich um einen Titankomplex mit Diethyltartrat als zweizähnigem chiralen Liganden. Das Verfahren weist jedoch den Nachteil auf, dass es sehr spezielle Reaktionsbedingungen benötigt. So muss die Umsetzung in der Regel in Gegenwart einer Base und in einer ganz bestimmten Reihenfolge durchgeführt werden. Beispielsweise muss der Titankomplex in Gegenwart des prochiralen Sulfids umgesetzt werden, und die Umsetzung sollte bei erhöhter Temperatur und/oder einer erhöhten Umsetzungszeit erfolgen. Ferner wird in den Verfahren dieser Druckschrift ein sehr spezielles Oxidationsmittel, nämlich Cumolhydroperoxid, eingesetzt.

Ein neueres Verfahren zur asymmetrischen Synthese von Benzimidazolderivaten mit einer chiralen Sulfoxidgruppe ist in der WO 03/089408 beschrieben. Diese Umsetzung erfolgt in Gegenwart einer Base mit einem Titan- oder Vanadiumkatalysator mit einem chiralen einzähnigen Liganden.

Allgemein sind im Stand der Technik eine Vielzahl von Verfahren zur asymmetrischen Oxidation von Sulfiden zu optisch aktiven Sulfoxiden bekannt, und es kann beispielsweise auf die Druckschrift Journai of Organic Chemistry 1998, 63, 9392-9395 verwiesen werden. Ob eines der vielen allgemein beschriebenen Verfahren zur asymmetrischen Oxidation von Sulfiden zu den optisch aktiven Sulfoxiden auch zur Herstellung der gewünschten substituierten Benzimidazole mit einer chiralen Sulfoxidgruppe geeignet ist und gegenüber den bekannten Verfahren Vorteile mit sich bringt, kann aber nicht vorhergesagt werden. Dies gilt insbesondere für die asymmetrische Synthese der einzelnen Enantiomere des Omeprazols, insbesondere des Esomeprazols.

Es besteht daher ein Bedarf nach weiteren Verfahren zur Herstellung einzelner Enantiomere von Benzimidazolderivaten mit einer chiralen Sulfoxidgruppe, die die Nachteile des Standes der Technik nicht zeigen.

Die Erfindung stellt ein Verfahren zur Herstellung eines optisch aktiven Enantiomers oder einer enantiomerenangereicherten Form einer Verbindung der Formel (I) 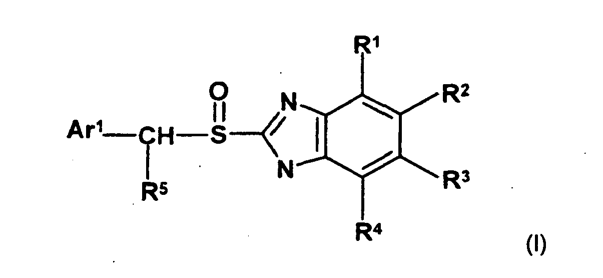 zur Verfügung, in der die Reste R¹, R², R³ und R⁴ unabhängig voneinander Wasserstoff, Alkyl, Alkoxy, Halogen, Halogenalkoxy, Alkylcarbonyl, Alkoxycarbonyl, Oxazolyl oder Halogenalkyl sind oder benachbarte Reste R¹, R², R³ und R⁴ gegebenenfalls substituierte Ringstrukturen ausbilden, R⁵ ein Wasserstoffatom darstellt oder mit dem Rest Ar¹ zu einem kondensierten Ringsystem verbunden ist und Ar¹ ein Rest der Formel 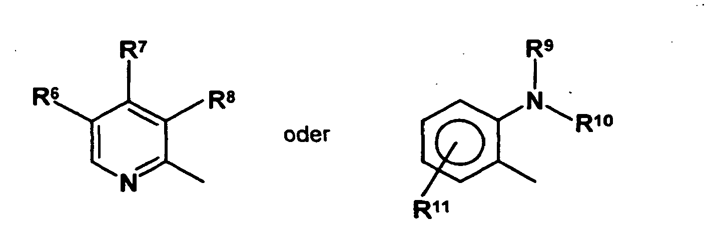 darstellt, in der die Reste R⁶, R⁷ und R⁸ unabhängig voneinander Wasserstoff, Alkyl, Alkylthio, Alkoxy, Halogen substituiertes Alkoxy, Alkoxyalkoxy, Dialkylamino, Piperidino, Morpholino, Halogen, Phenylalkyl oder Phenylalkoxy sind oder einer dieser Reste mit dem Rest R⁵ zu einem kondensierten Ringsystem verbunden ist, die Reste R⁹ und R¹⁰ unabhängig voneinander Wasserstoff, Halogen oder Alkyl darstellen und der Rest R¹¹ Wasserstoff, Halogen, Trifluormethyl, Alkyl oder Alkoxy ist.

Bei dem erfindungsgemäßen Verfahren wird ein prochirales Sulfid der Formel (II) 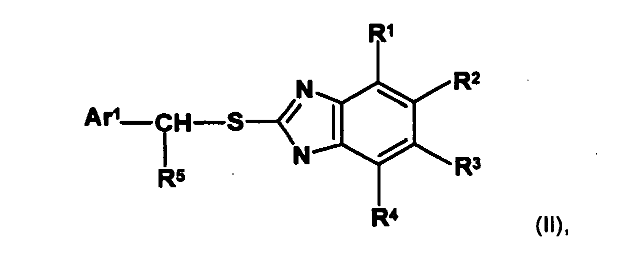 bei der die Reste R¹, R², R³, R⁴, R⁵ und Ar¹ wie vorstehend definiert sind, in einem organischen Lösungsmittel mit einem Oxidationsmittel in Gegenwart eines Katalysators oxidiert. Das Verfahren ist **dadurch gekennzeichnet, dass** der Katalysator ein Titan-(IV)-komplex ist, der durch Umsetzen einer Titan-(IV)-verbindung mit einem chiralen zweizähnigen (R,R)- oder (S,S)-1,2-bis-Arylethan-1,2-diol bei einer Temperatur im Bereich von 20°C bis 25°C über einen Zeitraum vom 10 bis 20 Minuten erhältlich ist, und wobei die katalytische Oxidation durchgeführt wird, ohne dass eine Base zugesetzt wird.

Bei dem chiralen zweizähnigen (R,R)- oder (S,S)-1,2-bis-Arylethan-1,2-diol handelt es sich bevorzugt um eine Verbindung der allgemeinen Formel (III) oder (III') 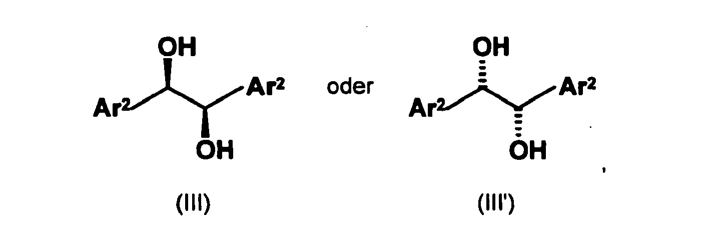 in der der Rest Ar² ausgewählt ist aus 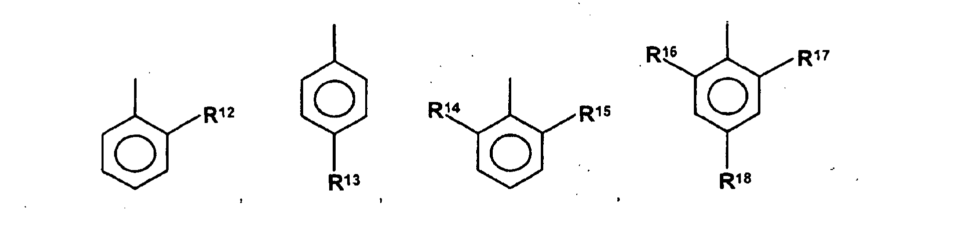 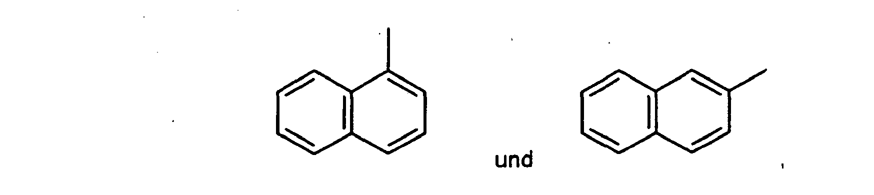 wobei die Reste R¹² bis R¹⁸ unabhängig voneinander ausgewählt sind aus Wasserstoff, Alkyl, Alkoxy, Carbonsäureesterrest, Halogen, Phenyl, Trifluormethyl und NO₂.

Im Rahmen der vorliegenden Anmeldung bedeutet Alkyl bevorzugt einen C₁-C₂₀-, bevorzugt einen C₁-C₁₀, stärker bevorzugt einen C₁-C₆-Alkylrest wie eine Methyl-, Ethyl-, Isopropyl-, n-Propyl-, n-Butyl-, Isobutyl- und tert.-Butylgruppe.

Im Rahmen der vorliegenden Anmeldung bedeutet Alkoxy bevorzugt einen Alkoxyrest mit 1 bis 20, stärker bevorzugt mit 1 bis 10 und insbesondere mit 1 bis 6 Kohlenstoffatomen wie eine Methoxy-, Ethoxy, Isopropoxy-, n-Propoxy, n-Butoxy-, Isobutoxy- und tert.-Butoxygruppe.

Im Rahmen dieser Anmeldung bedeutet Halogen ein Halogenatom, insbesondere ein Fluor-, Chlor-, Brom- oder Iodatom, wobei Fluoratome besonders bevorzugt sind.

Im Rahmen dieser Anmeldung bedeutet Halogenalkoxy bevorzugt Alkoxy wie vorstehend definiert, das mit einem oder mehreren, insbesondere 1 bis 5, stärker bevorzugt 1 bis 3, insbesondere 1, 2 oder 3 Halogenatomen wie vorstehend definiert substituiert ist. Die Halogenatome können gleich oder verschieden sein und an einem oder mehreren Kohlenstoffatomen sitzen. Bevorzugt sind die Halogenatome gleich und sind (soweit dies chemisch möglich ist) an das gleiche Kohlenstoffatom gebunden, wie z.B. bei einer CF₃-Gruppe.

Im Rahmen dieser Anmeldung bedeutet Halogenalkyl bevorzugt Alkyl wie vorstehend definiert, das mit einem oder mehreren, insbesondere 1 bis 5, stärker bevorzugt 1 bis 3, insbesondere 1, 2 oder 3, Halogenatomen wie vorstehend definiert substituiert ist. Die Halogenatome können gleich oder verschieden sein und an einem oder mehreren Kohlenstoffatomen sitzen. Bevorzugt sind die Halogenatome gleich und sind (soweit dies chemisch möglich ist) an das gleiche Kohlenstoffatom gebunden, wie z.B. bei einer CF₃-Gruppe.

Im Rahmen dieser Anmeldung bedeutet ein Alkylcarbonylrest bevorzugt Alkyl wie vorstehend definiert, das eine Carbonylfunktionalität C=O aufweist.

Im Rahmen dieser Anmeldung bedeutet Alkoxycarbonyl bevorzugt Alkoxy wie vorstehend definiert, das eine Carbonylgruppe, C=O, aufweist.

Aryl im Rahmen dieser Anmeldung ist bevorzugt eine Phenyl- oder 1- oder 2-Naphthylgruppe. Aryl kann gegebenenfalls mit ein bis drei Substituenten substituiert sein, insbesondere mit Halogenatomen, Nitro, Alkyl und Alkoxy wie vorstehend definiert.

Im Rahmen dieser Anmeldung ist Alkylthio bevorzugt Alkyl wie vorstehend definiert, das eine Thiogruppe aufweist.

Im Rahmen dieser Anmeldung ist Alkoxyalkoxy bevorzugt Alkoxy wie vorstehend definiert, der mit einem Alkoxy wie vorstehend definiert substituiert ist.

Im Rahmen dieser Anmeldung ist Dialkylamino eine Aminogruppe, die mit zwei Alkyl wie vorstehend definiert substituiert ist.

Im Rahmen dieser Anmeldung ist Phenylalkyl bzw. Phenylalkoxy Alkyl bzw. Alkoxy wie vorstehend definiert, die mit einer Phenylgruppe substituiert sind.

Im Rahmen dieser Anmeldung ist ein Carbonsäureesterrest bevorzugt der Rest einer Carbonsäure mit 1 bis 10 Kohlenstoffatomen, bevorzugt mit 1 bis 6 Kohlenstoffatomen, stärker bevorzugt mit 1 bis 4 Kohlenstoffatomen.

im Rahmen dieser Anmeldung ist Arylalkyl bevorzugt Alkyl wie vorstehend definiert, das mit einem Aryl wie vorstehend definiert substituiert ist.

Sofern Reste im Rahmen der vorliegenden Anmeldung Ringstrukturen oder kondensierte Ringsysteme ausbilden können, schließen diese Reste bevorzugt einen Kohlenstoffring mit 5 bis 10 Kohlenstoffatomen, bevorzugt mit 5, 6 oder 7 Kohlenstoffatomen, der gegebenenfalls substituierte sein kann.

Sofern im Rahmen dieser Anmeldung eine Einheit substituiert sein kann, ist sie bevorzugt mit einem Halogenatom einer C₁-C₆-Alkylgruppe oder einer C₁-C₆-Alkoxygruppe wie vorstehend definiert substituiert, sofern nichts anderes angegeben ist.

Erfindungsgemäß besonders bevorzugt handelt es sich bei der Verbindung der Formel (I) um eine Verbindung der Formel 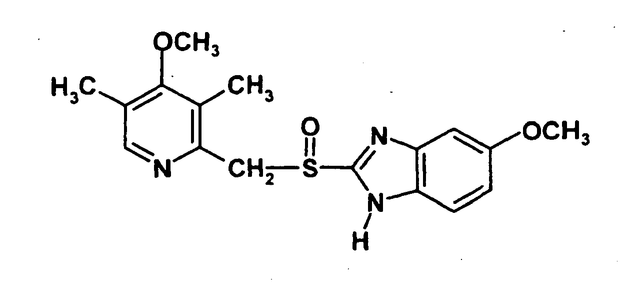 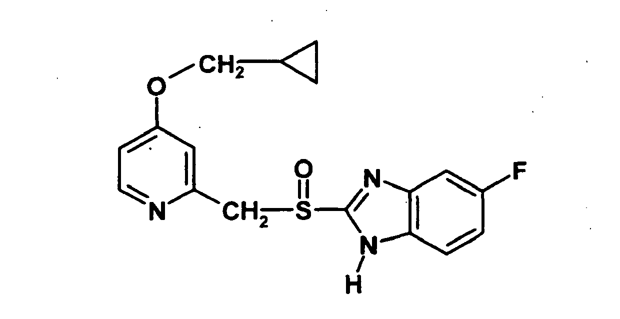 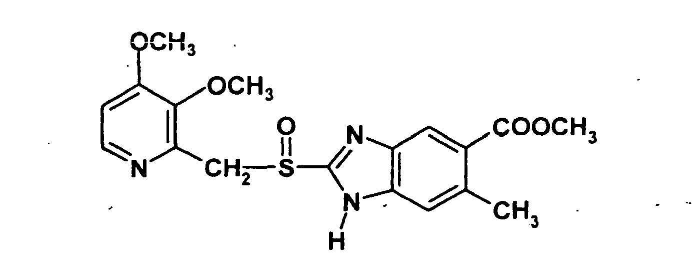 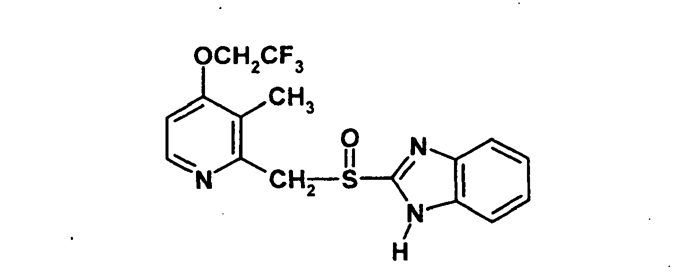 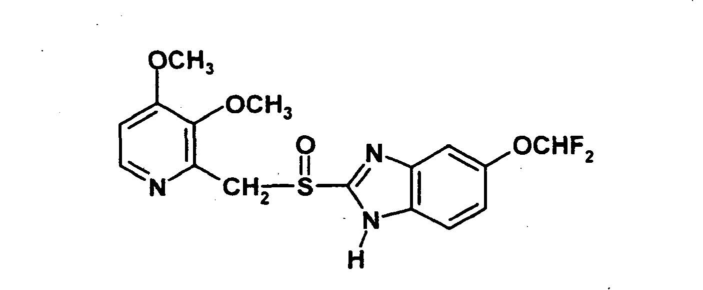 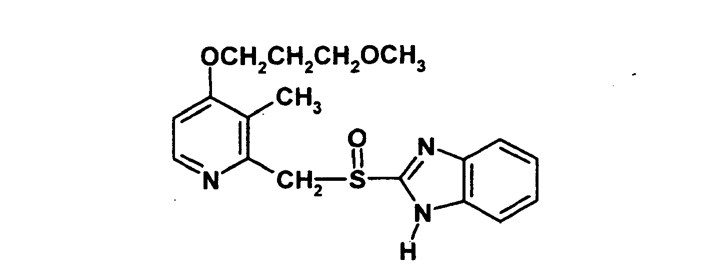 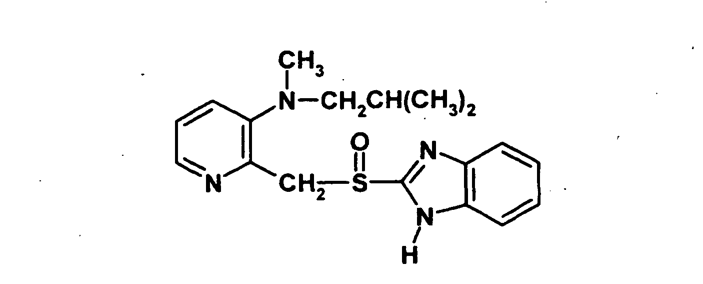 oder

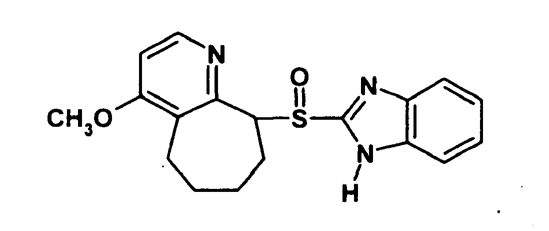

Am stärksten bevorzugt handelt es sich bei der Verbindung der Formel (I) erfindungsgemäß um Omeprazol, Pantoprazol, Lansoprazol oder Rabeprazol. Omeprazol ist am stärksten bevorzugt. In einer besonders bevorzugten Ausführungsform betrifft daher die Erfindung ein Verfahren zur Herstellung eines Enantiomeren des Omeprazols und der Salze davon oder eines Gemischs der beiden Enantiomeren des Omeprazols, in dem ein Enantiomer in einem höheren Anteil vorkommt als das andere Enantiomer. Insbesondere betrifft die Erfindung ein Verfahren zur Herstellung von Esomeprazol und dessen Salzen, insbesondere das Zinksalz des Esomeprazols.

Im folgenden wird die Erfindung beispielhaft im wesentlichen anhand von Omeprazol weiter erläutert. Die nachstehenden Ausführungen gelten aber gleichermaßen für die übrigen Verbindungen der Formel (I) vorstehend.

In den Resten Ar² ist der Rest R¹² besonders bevorzugt Alkyl wie vorstehend definiert oder Halogen wie vorstehend definiert, insbesondere ein Bromatom.

Der Rest R¹³ ist insbesondere Alkyl wie vorstehend definiert, Halogen wie vorstehend definiert, insbesondere ein Bromatom oder Alkoxy wie vorstehend definiert, besonders bevorzugt ein Alkylrest mit 1 bis 4 Kohlenstoffatomen oder ein Bromatom.

Die Reste R¹⁴ und R¹⁵ sind bevorzugt gleich und ausgewählt aus einem Wasserstoffatom, Halogen, Alkyl und Alkoxy wie vorstehend definiert.

Die Reste R¹⁶, R¹⁷ und R¹⁸ sind ebenfalls bevorzugt gleich und stärker bevorzugt ein Wasserstoffatom oder Alkyl wie vorstehend definiert.

Besonders bevorzugt ist das (R,R)- oder (S,S)-1,2-bis-Arylethyl-1,2-diol, die Verbindung

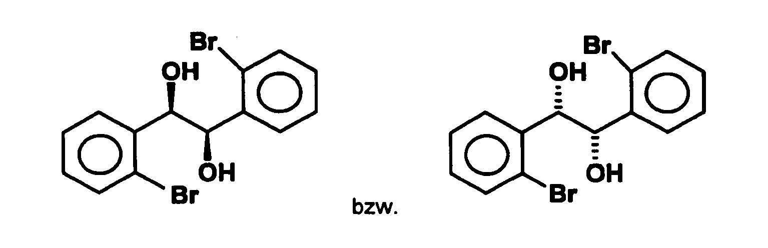

Das (R,R)- oder (S,S)-1,2-bis-Arylethyl-1,2-diol, das erfindungsgemäß als chiraler Ligand der Titanverbindung eingesetzt wird, kann auf an sich bekannte Art und Weise hergestellt werden bzw. ist kommerziell erhältlich. Insbesondere können diese Verbindungen durch asymmetrische Dihydroxylierung von (E)-Stilben bzw. der entsprechenden Stilbenderivate erhalten werden, wie es beispielsweise in Chem. Rev. 1994, 94, 2483 oder Chirality 13: 258-265 (2001) beschrieben ist. Auf beide Druckschriften wird hinsichtlich der Herstellung der Diole vollinhaltlich Bezug genommen.

Erfindungsgemäß wird der Katalysator in situ gebildet durch Umsetzung einer geeigneten Titanverbindung, insbesondere eines Titan-(IV)-alkoxids, bevorzugt eines Titan-(IV)-isopropoxids (Ti(i-PrO)₄) mit dem entsprechenden chiralen Diol der Formel (III) bzw. (III'). Die Umsetzung der Titan-(IV)-verbindung mit dem Diol erfolgt in einem organischen Lösemittel bevorzugt in Gegenwart von Wasser, und bevorzugt wird zur Herstellung des Katalysators das gleiche Lösemittel verwendet, das auch später für die Oxidation verwendet wird. Hierbei handelt es sich insbesondere um Halogen-substituierte oder unsubstituierte Alkyl- und Arylkohlenwasserstoffe wie Methylenchlorid, Chloroform, Kohlenstofftetrachlorid, Hexan und Toluol. Das am stärksten bevorzugte organische Lösemittel ist Toluol. Die Herstellung des Katalysators erfolgt bei einer Temperatur im Bereich von 20°C bis 25°C. Die Herstellung des Katalysators erfolgt über einen Zeitraum von 10 bis 20 Minuten.

Bevorzugt wird erfindungsgemäß zunächst in situ der Katalysator hergestellt und anschließend wird zu dem Reaktionsgemisch, das den Katalysator enthält, das prochirale Sulfid der Formel (II), insbesondere die Verbindung 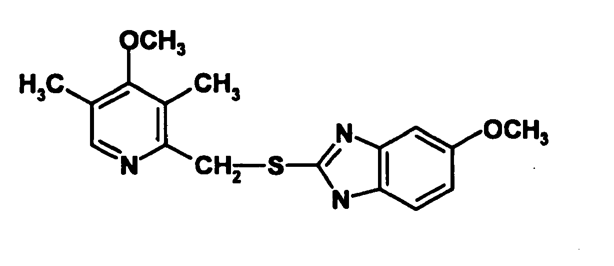 gegeben, und anschließend wird das Oxidationsmittel zugegeben.

Alternativ ist es ebenfalls bevorzugt, den Katalysator in Gegenwart des prochiralen Sulfids der Formel (II) herzustellen, d.h. dass z.B. zunächst die Titanverbindung vorgelegt wird, anschließend das prochirale Sulfid der Formel (II) zugegeben wird und anschließend das chirale Diol zugegeben wird. Auch hier wird das Oxidationsmittel bevorzugt als letztes zugegeben.

Die anschließende Oxidation erfolgt bevorzugt in dem gleichen Lösemittelgemisch, in dem auch der Katalysator hergestellt wurde, das heißt auch in Gegenwart von Wasser, in einem organischen Lösemittel, das bevorzugt ausgewählt ist aus Halogen-substituierten oder unsubstituierten Alkyl- und Arylkohlenwasserstoffen wie Methylenchlorid, Chloroform, Kohlenstofftetrachlorid, Hexan und Toluol, bevorzugt Toluol.

Die enantioselektive katalytische Reaktion wird bevorzugt bei einer Temperatur im Bereich von -78°C bis 25°C, stärker bevorzugt bei etwa -20°C bis 0°C, insbesondere bei etwa -20°C, durchgeführt. Die Dauer der enantioselektiven katalytischen Oxidation ist in der Regel 2 bis 24 Stunden, bevorzugt 12 bis 18 Stunden.

Jedes übliche Oxidationsmittel kann erfindungsgemäß verwendet werden, besonders bevorzugt ist das Oxidationsmittel jedoch Wasserstoffperoxid, ein Alkylhydroperoxid oder ein Arylalkylhydroperoxid, wobei tert.-Butylhydroperoxid besonders bevorzugt ist. Bevorzugt ist das Oxidationsmittel nicht Cumolhydroperoxid.

Die erfindungsgemäße enantioselektive katalytische Oxidation wird durchgeführt, ohne dass eine Base zugesetzt wird.

Bei dem erfindungsgemäßen Verfahren werden die Mengen an Katalysator und prochiralem Sulfid der Formel (II) bevorzugt so gewählt, dass das Molverhältnis des chiralen zweizähnigen Liganden zu dem prochiralen Sulfid der Formel (II) im Bereich von 0,02:1 bis 0,4:1 liegt, stärker bevorzugt etwa 0,1:1 ist. Erfindungsgemäß bevorzugt ist das Molverhältnis der Titanverbindung zu dem Sulfid der Formel (II) im Bereich von 0,01:1 bis 0,2:1, stärker bevorzugt etwa 0,05:1.

Erfindungsgemäß bevorzugt wird die Herstellung des Katalysators sowie auch die folgende enantioselektive katalytische Oxidation in Gegenwart von Wasser durchgeführt, wobei bevorzugt das Molverhältnis des Wassers zu dem prochiralen Sulfid der Formel (II) im Bereich von 0,01:1 bis 2:1 liegt, stärker bevorzugt etwa 1:1 ist.

Die Menge an verwendetem Oxidationsmittel ist nicht kritisch, bevorzugt ist das Verhältnis des Oxidationsmittels zu dem prochiralen Sulfid der Formel (II) im Bereich von 0.5:1 bis 3:1, bevorzugt etwa 2:1.

Die Aufarbeitung des Reaktionsgemischs nach der Durchführung der enantioselektiven Oxidation des prochiralen Sulfids der Formel (II) zu dem chiralen Sulfoxid der Formel (I) ist nicht weiter kritisch, allerdings wurde gefunden, dass nach einem speziellen Aufarbeitungsverfahren das Sulfoxid der Formel (I), insbesondere das Esomeprazol, in der basischen Form anfällt, die anschließend besonders leicht in ihre Salze überführt werden kann.

Bevorzugt wird erfindungsgemäß das Reaktionsgemisch nach Durchführung der enantioselektiven katalytischen Oxidation der Verbindung der Formel (II) zu der Verbindung der Formel (I) mit einer wässrigen basischen Lösung behandelt. Bei der wässrigen basischen Lösung handelt es sich bevorzugt um eine wässrige Ammoniaklösung. Nach Zugabe der Ammoniaklösung wird eine Säure zugegeben, bei der es sich um die wässrige Lösung einer anorganischen Säure oder um eine organische Säure handeln kann, bevorzugt ist eine organische Säure und besonders bevorzugt ist Essigsäure. Es wird ein pH-Wert bevorzugt im Bereich von 5 bis 8 eingestellt, besonders bevorzugt ein pH-Wert im Bereich von 6 bis 7,5. Die so erhaltene wässrige Lösung wird mit einem organischen Lösemittel extrahiert, wobei Halogen-substituierte oder unsubstituierte Alkyl- oder Arylkohlenwasserstoffe und Ketone wie Methylenchlorid, Chloroform, Kohlenstofftetrachlorid, Hexan, Toluol, Aceton, Butanon und Methylisobutylketon als besonders bevorzugt genannt werden können. Auch andere übliche organische Extraktionsmittel können verwendet werden. Das bevorzugte Lösemittel für die Extraktion ist Methylisobutylketon.

Überraschend wurde gefunden, dass die Verbindung der Formel (I), insbesondere das (S)-Omeprazol (Esomeprazol), durch Kühlen des organischen Extraktionsmittels rein ausfällt. Bevorzugt wird die Extraktionslösung auf eine Temperatur in einem Bereich von -78°C bis 25°C, stärker bevorzugt in einem Bereich von -20°C bis 0°C, z.B. etwa -10°C gekühlt, und das gewünschte Enantiomer der Verbindung der Formel (I) fällt als Feststoff in Form der freien Base aus.

Auf diese Art und Weise kann insbesondere das S-Enantiomer des Omeprazols in sehr guter Ausbeute und optischer Reinheit auf einfache Art und Weise gewonnen werden. Falls das so erhaltene Produkt noch Reste des unerwünschten Enantiomers der Verbindung der Formel (I) aufweist, können diese auf übliche Art und Weise zur Erhöhung der optischen Reinheit abgetrennt werden.

In der Regel fällt das gewünschte Enantiomer als Feststoff als Gemisch aus amorphem und kristallinem Produkt an, insbesondere bei der Herstellung des Esomeprazols.

Da bei dem erfindungsgemäßen Verfahren mit der bevorzugten Aufarbeitung das gewünschte Isomer der Verbindung der Formel (I) in Form der freien Base als Feststoff anfällt, kann es besonders vorteilhaft zu einem Salz umgesetzt werden. Dies ist ein Vorteil gegenüber den Verfahren des Standes der Technik, bei denen die Herstellung eines bestimmten Salzes des Esomeprazols nur aufwendig möglich ist, wie es z.B. in der WO 98/28294 beschrieben ist, nämlich durch Auflösen eines alkalischen Salzes des Esomeprazols in Wasser, Extraktion des neutralen Esomeprazols mit einem organischen Lösemittel durch Absenken des pH-Werts mit einer wasserlöslichen Säure, Abdampfen zu einer stark konzentrierten Lösung und Zugabe eines Nicht-Lösemittels, wodurch das Esomeprazol in basischer Form als Feststoff ausfällt. Nach dem erfindungsgemäßen Verfahren erhält man auf einfache Art und Weise direkt durch geeignete Aufarbeitung des Reaktionsgemischs nach der enantioselektiven katalytischen Oxidation des entsprechenden Sulfids zu dem Sulfoxid die freie Base als Feststoff. Die freie Base kann dann auf übliche Art und Weise in ein gewünschtes Salz überführt werden, wobei die Salze nicht besonders eingeschränkt sind. Hier können insbesondere das Natrium-, Magnesium-, Lithium-, Kalium-, Calcium- und das quaternäre Ammoniumsalz genannt werden, aber auch das Piperidinsalz und insbesondere das Zinksalz. Das Zinksalz des Esomeprazols kann erfindungsgemäß besonders bevorzugt hergestellt werden, indem man das Esomeprazol mit einer geeigneten Zinkquelle behandelt. Bevorzugte Zinkquellen sind Zinkacetat, Zinkbromid, Zinkcarbonathydroxid, Zinkchlorid, Zinktrifluormethansulfonat, Zinknitrat, Diethylzink und Zinksulfat, wobei Diethylzink und Zinkchlorid, insbesondere Diethylzink, besonders bevorzugt sind.

In der am stärksten bevorzugten Ausführungsform wird erfindungsgemäß daher ein Verfahren zur Herstellung von Esomeprazol oder eines Salzes des Esomeprazols, insbesondere des Zinksalzes des Esomeprazols, zur Verfügung gestellt, das die folgenden Schritte umfasst:
a) (R,R)-1,2-bis-Arylethyl-1,2-diol, insbesondere (R,R)-1,2-bis-(2-Bromphenyl)ethan-1,2-diol, wird mit einem Titan-(IV)-alkoxid, insbesondere mit Titantetraisopropoxid, in einem organischen Lösemittel vorgelegt;
b) zu diesem Reaktionsgemisch wird Wasser zugegeben;
c) zu dem Reaktionsgemisch des Schritts b) wird das entsprechende Sulfid der allgemeinen Formel (II), insbesondere die Verbindung 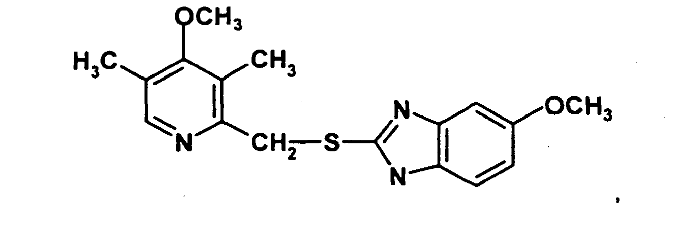 zugegeben;
d) zu diesem Gemisch wird das Oxidationsmittel, insbesondere ein Alkyl- oder Arylalkylhydroperoxid, am stärksten bevorzugt Dibutylhydroperoxid, zugegeben;
e) eine wässrige basische Lösung, insbesondere eine wässrige Ammoniaklösung, wird zugesetzt;
f) zu dem Gemisch wird eine Säure, insbesondere eine organische Säure wie Essigsäure, zugesetzt, bevorzugt bis zu einem pH-Wert im Bereich von 5 bis 8, besonders bevorzugt im Bereich von 6 bis 7,5;
g) das wässrige Gemisch wird mit einem geeigneten organischen Lösemittel extrahiert;
h) das organische Lösemittel wird gekühlt, und das ausgefallene Enantiomer der Verbindung der Formel (I), insbesondere das Esomeprazol, wird abfiltriert und
i) gegebenenfalls zu einem Salz, insbesondere einem Zinksalz, umgesetzt.

Die folgenden Beispiele erläutern die Erfindung.

### Beispiel 1

### Herstellung des S-Omeprazols

Titantetraisopropoxid (4,5 mg, 0,016 mmol) wurde zu einer Lösung aus (R,R)-1,2-bis-(2-Bromphenyl)ethan-1,2-diol (12 mg, 0,032 mmol) in Toluol (2 ml) bei 25°C gegeben. Die Lösung wurde 10 Minuten gerührt, Wasser (5,7 mg, 0,32 mmol) wurde zugegeben, und es wurde weitere 10 Minuten gerührt. 5-Methoxy-2-[[(4-methoxy-3,5-dimethyl-2-pyridinyl)-methyl]thio]-1H-benzimidazol (105 mg, 0,32 mol) wurde dann zu der Lösung gegeben, und die Temperatur wurde auf -20°C eingestellt. Anschließend wurde langsam t-Butylhydroperoxid (70%, 96 µl, 0,064 mmol) zugegeben. Nach 12 Stunden bei -20°C wurde die Lösung dreimal mit wässrigem Ammoniumhydroxid (12,5% NH₃, 3x5 ml) extrahiert. Anschließend wurde Methylisobutylketon (5 ml) zu den vereinigten wässrigen Extrakten gegeben. Danach wurde der pH-Wert der wässrigen Phase mit Essigsäure eingestellt, die wässrige Phase wurde abgetrennt und mit einer zusätzlichen Menge Methylisobutylketon (5 ml) extrahiert. Die organische Lösung wurde über Nacht auf -10°C gekühlt, und die neutrale Form des S-Omeprazols in fester Form wurde ausgefällt, um die Titelverbindung zu erhalten (99 mg, 90% Ausbeute). Der enantiomere Überschuss des S-Omeprazols betrug 94%. Auf Reinigung mit Methytisobutylketon ergab das S-Omeprazol, der enantiomere Überschuss war >99%.

### Beispiel 2

### Herstellung des R-Omeprazols

Titantetraisopropoxid (4,5 mg, 0,016 mmol) wurde zu einer Lösung aus (S,S)-1,2-bis-(2-Bromphenyl)ethan-1,2-diol (12 mg, 0,032 mmol) in Toluol (2 ml) bei 25°C gegeben. Die Lösung wurde 10 Minuten gerührt, Wasser (5,7 mg, 0,32 mmol) wurde zugegeben, und es wurde weitere 10 Minuten gerührt. 5-Methoxy-2-[[(4-methoxy-3,5-dimethyl-2-pyridinyl)-methyl]thio]-1H-benzimidazol (105 mg, 0,32 mol) wurde dann zu der Lösung gegeben, und die Temperatur wurde auf -20°C eingestellt. Anschließend wurde langsam t-Butylhydroperoxid (70%, 96 µl, 0,064 mmol) zugegeben. Nach 12 Stunden bei -20°C wurde die Lösung dreimal mit wässrigem Ammoniumhydroxid (12,5% NH₃, 3x5 ml) extrahiert. Anschließend wurde Methylisobutylketon (5 ml) zu den vereinigten wässrigen Extrakten gegeben. Danach wurde der pH-Wert der wässrigen Phase mit Essigsäure eingestellt, die wässrige Phase wurde abgetrennt und mit einer zusätzlichen Menge Methylisobutylketon (5 ml) extrahiert. Die organische Lösung wurde über Nacht auf -10°C gekühlt, und die neutrale Form des R-Omeprazols in fester Form wurde ausgefällt, um die Titelverbindung zu erhalten. Der enantiomere Überschuss des R-Omeprazols betrug 93%.

### Beispiel 3

### Herstellung von Esomeprazolzink

Esomeprazol (1 g, 2,9 mmol) wurde in 10 ml Tetrahydrofuran unter 5-stündigem Rühren gelöst, und 2,9 ml Diethylzink (1 M Lösung in Hexan) wurden langsam zugegeben. Das erhaltene Gemisch wurde über Nacht bei Umgebungstemperatur gerührt. 10 ml destilliertes Wasser wurden zugegeben, und das gebildete Präzipitat wurde abfiltriert und mit destilliertem Wasser gewaschen. Man erhielt 1 g (91%) der Titelverbindung.

### Beispiel 4 (Herstellung des Katalysatorliganden)

### (E)-2,2-Dibfomstilben

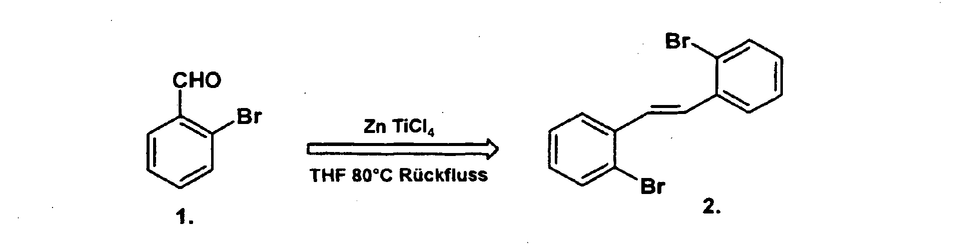

4,4 ml (7,4 g 40 mmol) einer gelben Aufschlämmung aus Titan-(IV)-chlorid in 150 ml Tetrahydrofuran wurde im Eisbad unter Stickstoff mit einem Magnetrührer gerührt. 5g (77 mmol) Zinkstaub wurden vorsichtig zugegeben. Dann wurden 7 g (38 mmol) Aldehyd 1 in 50 ml Tetrahydrofuran zugegeben, und die Mischung wurde 8 Stunden lang unter Rückfluss erhitzt. Die abgekühlte Reaktionsmischung wurde in 1M verdünnte wässrige Salzsäure gegossen und das Produkt wurde mit Hexan extrahiert. Die kombinierten Extrakte wurden mit Wasser und (Koch-)Salzlösung gewaschen, mit Natriumsulfat getrocknet, filtriert und das Filtrat wurde rotationsverdampft, wobei 6,2 g (97 %) von 2. erhalten wurden. Durch Umkristallisation aus einer Mischung aus 5 % Toluol und 95 % Ethanol erhielt man 2. als weiße Nadeln. (1R, 2R)-1, 2-Bis(2-bromphenyl)ethan-1,2-diol

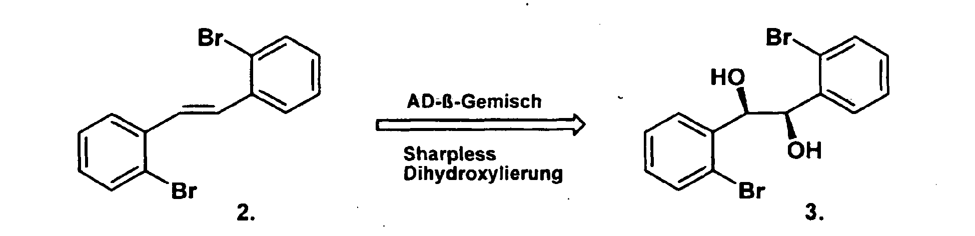

Methansulfonamid (3,39 g, 0,0419 mol) und AD-mix-β (50,2 g) wurden in einen 1 Liter Dreihalskolben gegeben, in dem Wasser (180 ml) und 2-Methylpropan-2ol (180 ml) vorgelegt worden waren. Die Mischung wurde mit einem mechanischen Rührer so lange gerührt, bis alle Feststoffe gelöst waren. Der Kolben wurde dann auf 0°C gekühlt und es wurde Dibromstilben **2** (12,0 g, 32,3 mmol) dazugegeben. Die Reaktionsmischung wurde 72 Stunden lang stark gerührt und zwischen 0 und 3°C gehalten. Dann wurde wasserfreies Natriumsulfid (54 g, 0,439 mol) dazugegeben und die Mischung wurde über Nacht gerührt. Dichlormethan (350 ml) wurde hinzugefügt und die Phasen wurden getrennt. Die wässrige Schicht wurde mit Dichlormethan (3 x 175 ml) extrahiert und die vereinigten organischen Schichten wurden mit 2 M KOH (30 ml) gewaschen, getrocknet (MgSO₄) und flüchtige Stoffe wurden unter verringertem Druck abgedampft. Der Rückstand wurde mit Blitzchromatographie durch Eluieren mit Ether-Hexan gereinigt und dann umkristallisiert (Hexan-Dichlormethan, 1,1 : 1, 92 ml) wobei man das Diol **3** (12,5 g, 94 %) als Nadeln erhielt.

## Patentansprüche

1. Verfahren zur Herstellung eines optisch aktiven Enantiomers oder einer enantiomerenangereicherten Form einer Verbindung der Formel (I) 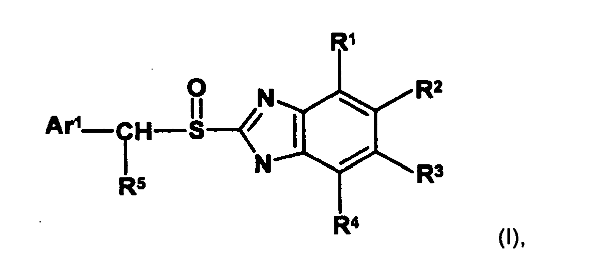 in der die Reste R¹, R², R³ und R⁴ unabhängig voneinander Wasserstoff, Alkyl, Alkoxy, Halogen, Halogenalkoxy, Alkylcarbonyl, Alkoxycarbonyl, Oxazolyl oder Halogenalkyl sind oder benachbarte Reste R¹, R², R³ und R⁴ gegebenenfalls substituierte Ringstrukturen ausbilden, R⁵ ein Wasserstoffatom darstellt oder mit dem Rest Ar¹ zu einem kondensierten Ringsystem verbunden ist und Ar¹ ein Rest der Formel 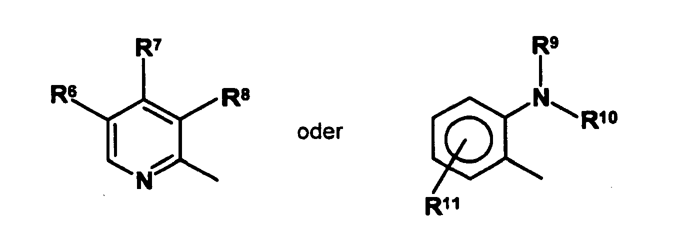 darstellt, in der die Reste R⁶, R⁷ und R⁸ unabhängig voneinander Wasserstoff, Alkyl, Alkylthio, Alkoxy, mit einem Halogen substituiertes Alkoxy, Alkoxyalkoxy, Dialkylamino, Piperidino, Morpholino, Halogen, Phenylalkyl oder Phenylalkoxy sind oder einer dieser Reste mit dem Rest R⁵ zu einem kondensierten Ringsystem verbunden ist, die Reste R⁹ und R¹⁰ unabhängig voneinander Wasserstoff, Halogen oder Alkyl darstellen und der Rest R¹¹ Wasserstoff, Halogen, Trifluormethyl, Alkyl oder Alkoxy ist, bei dem ein prochirales Sulfid der Formel (II) 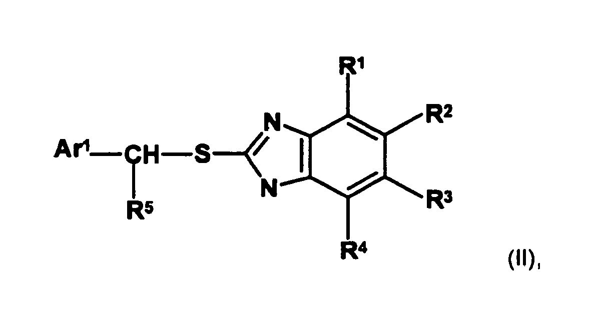 in der die Reste R¹, R², R³, R⁴, R⁵ und Ar¹ wie vorstehend definiert sind, in einem organischen Lösungsmittel mit einem Oxidationsmittel in Gegenwart eines Katalysators oxidiert wird, **dadurch gekennzeichnet, dass** der Katalysator ein Titan-(IV)-komplex ist, der durch Umsetzung einer Titan-(IV)-verbindung mit einem chiralen zweizähnigen (R,R)- oder (S,S)-1,2-bis-Arylethan-1,2-diol bei einer Temperatur im Bereich von 20 °C bis 25 °C über einen Zeitraum von 10 bis 20 Minuten erhältlich ist, und wobei die katalytische Oxidation durchgeführt wird, ohne dass eine Base zugesetzt wird.

2. Verfahren nach Anspruch 1, **dadurch gekennzeichnet, dass** es sich bei der Verbindung der Formel (I) um eine Verbindung der Formel 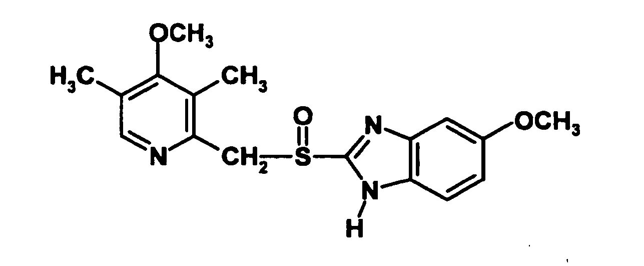 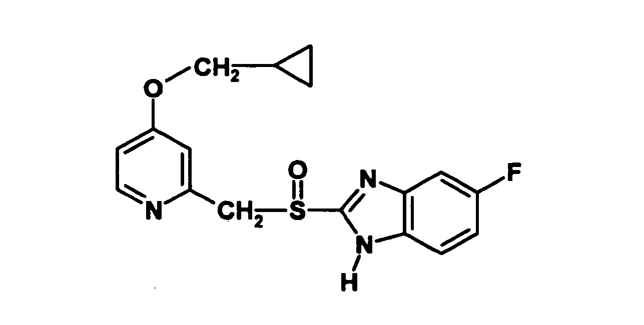 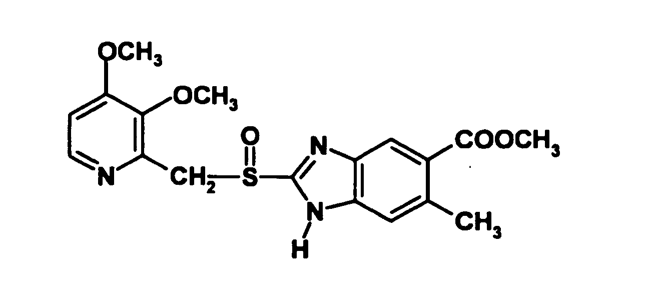 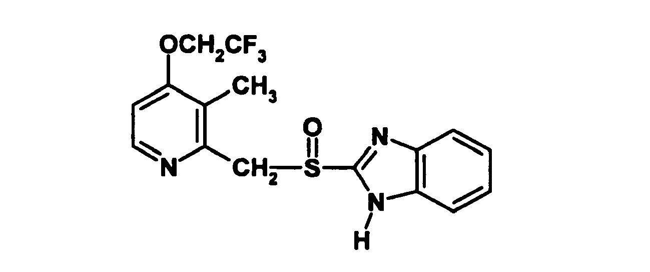 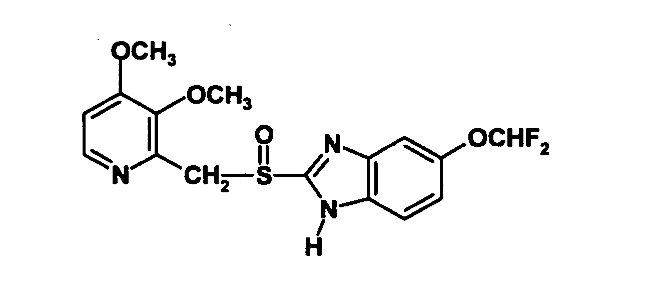 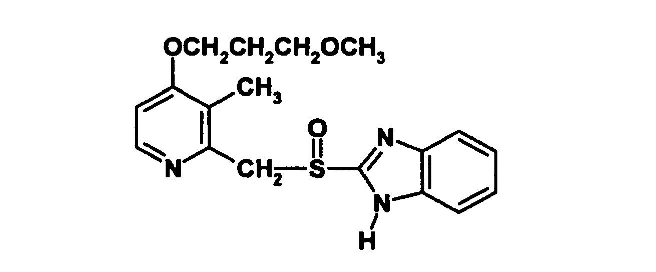 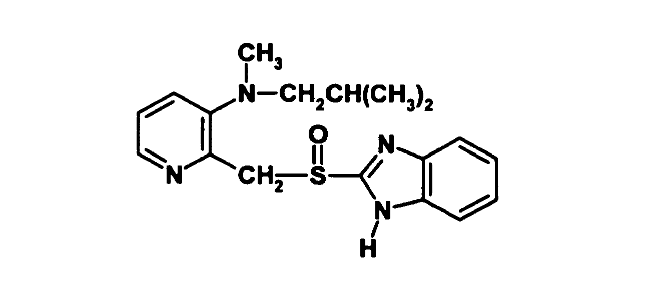 oder 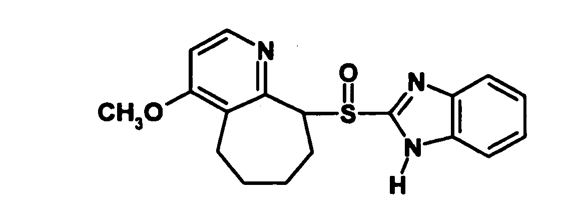 handelt und die Verbindung der Formel (II) das entsprechende prochirale Sulfid darstellt.

3. Verfahren nach Anspruch 2 zur Herstellung des S-Enantiomers des Omeprazols oder eines Gemischs aus dem S- und dem R-Enantiomer des Omeprazols, in dem das S-Enantiomer des Omeprazols angereichert ist.

4. Verfahren nach Anspruch 3, das den weiteren Schritt umfasst, dass das S-Enantiomer des Omeprazols mit einer Zinkquelle zu dem Zinksalz des S-Enantiomers des Omeprazols umgesetzt wird.

5. Verfahren nach einem der Ansprüche 1 bis 4, wobei es sich bei dem chiralen zweizähnigen (R,R)- oder (S,S)-1,2-bis-Arylethan-1,2-diol um eine Verbindung der allgemeinen Formel (III) oder (III') 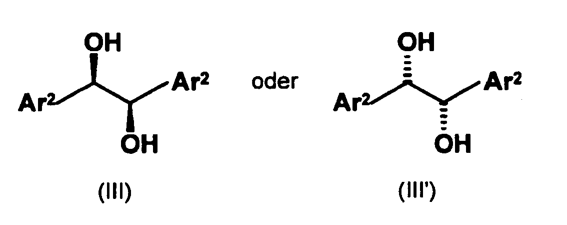 handelt, in der der Rest Ar² ausgewählt ist aus 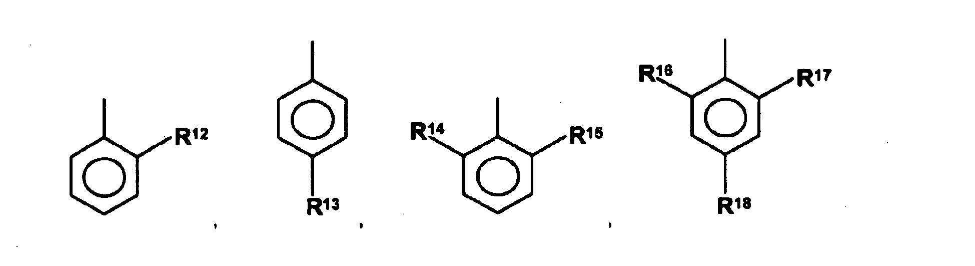 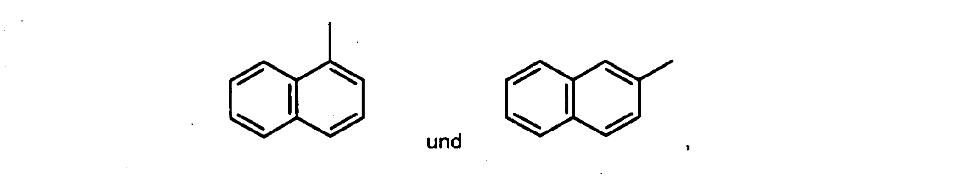 wobei die Reste R¹² bis R¹⁸ unabhängig voneinander ausgewählt sind aus Wasserstoff, Alkyl, Alkoxy, Carbonsäureesterrest, Halogen, Phenyl, Trifluormethyl und NO₂.

6. Verfahren nach Anspruch 5, wobei R¹⁴ und R¹⁵ unabhängig voneinander aus Wasserstoff, Alkyl, Alkoxy und Halogen ausgewählt sind und die Reste R¹⁶, R¹⁷ und R¹⁸ unabhängig voneinander aus Wasserstoff und Alkyl ausgewählt sind.

7. Verfahren nach Anspruch 6, wobei die Reste R¹⁴ und R¹⁵ gleich sind und die Reste R¹⁶, R¹⁷ und R¹⁸ gleich sind.

8. Verfahren nach Anspruch 7, wobei der Rest R¹² ein Bromatom darstellt.

9. Verfahren nach Anspruch 8, in dem das (R,R)- oder (S,S)-1,2-bis-Aryl-1,2-diol eine Verbindung der Formel 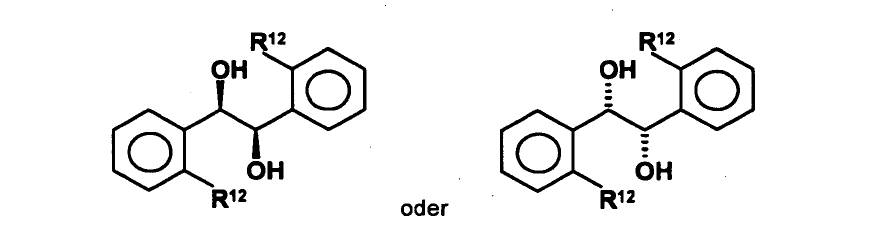 ist.

10. Verfahren nach einem der Ansprüche 1 bis 9, wobei die Titan-(IV)-verbindung ein Alkoxid des Titan-(IV) ist.

11. Verfahren nach Anspruch 10, wobei die Titanverbindung das Isopropoxid des Titan-(IV) ist.

12. Verfahren nach einem der vorstehenden Ansprüche, wobei das Verhältnis des chiralen zweizähnigen Liganden zu dem prochiralen Sulfid der Formel (II) im Bereich von 0,1:1 liegt.

13. Verfahren nach einem der vorstehenden Ansprüche, wobei das molare Verhältnis des Titan-(IV)-alkoxids zu dem prochiralen Sulfid der Formel (II) im Bereich von 0,05:1 liegt.

14. Verfahren nach einem der vorstehenden Ansprüche, wobei die Umsetzung in Gegenwart von Wasser durchgeführt wird.

15. Verfahren nach einem der vorstehenden Ansprüche, wobei das Oxidationsmittel Wasserstoffperoxid, ein Alkylhydroperoxid oder ein Arylalkylhydroperoxid ist.

16. Verfahren nach einem der vorstehenden Ansprüche, wobei der Katalysator durch Umsetzen des chiralen Liganden mit dem Titan-(IV)-alkoxid in einem organischen Lösemittel hergestellt wird, bevor das prochirale Sulfid der Formel (II) zu dem Reaktionsgemisch zugegeben wird.

17. Verfahren nach einem der vorstehenden Ansprüche, wobei die Oxidation bei etwa -20°C über einen Zeitraum im Bereich von 12 bis 18 Stunden durchgeführt wird.

18. Verfahren nach einem der vorstehenden Ansprüche, umfassend die folgenden Schritte:
a) Vorlegen eines Gemischs des chiralen zweizähnigen (R,R)- oder (S,S)-1,2-bis-Arylethan-1,2-diols mit dem Titan-(IV)-alkoxid in Gegenwart eines organischen Lösemittels,
b) Zugabe von Wasser zu dem Gemisch des Schritts a),
c) Zugabe des prochiralen Sulfids der Formel (II) zu dem Reaktionsgemisch des Schritts b),
d) Zugabe des Oxidationsmittels zu dem Reaktionsgemisch des Schritts c),
e) Zugabe von wässrigem Ammoniak zu dem Reaktionsgemisch des Schritts d),
f) Zugabe einer Säure zu dem wässrigen Gemisch des Schritts e),
g) Extraktion des wässrigen Gemischs mit einem organischen Lösemittel,
h) Abkühlen der organischen Lösung und Abfiltrieren des ausgefallenen Enantiomers der Verbindung der Formel (I) und
i) gegebenenfalls Überführen des gewünschten Isomers der Verbindung der Formel (I) in das Zinksalz.

## Claims

1. A process for the production of an optically active enantiomer or an enantiomer-enriched form of a compound of formula (I) 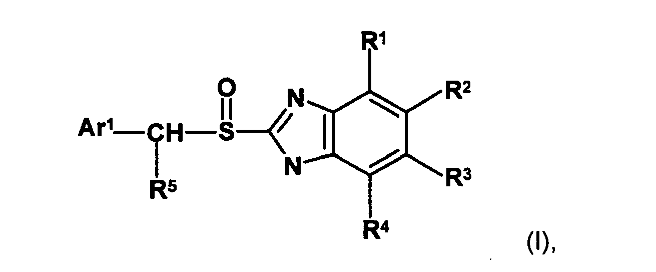 in which the residues R¹, R², R³ and R⁴ are independently hydrogen, alkyl, alkoxy, halogen, halogenalkoxy, alkylcarbonyl, alkoxycarbonyl, oxazolyl or halogenalkyl or adjacent residues R¹, R², R³ and R⁴ form ring structures, which are optionally subsituted, R⁵ represents a hydrogen atom or is connected with the residue Ar¹ to give a condensed ring system, and Ar¹ is a residue of formula 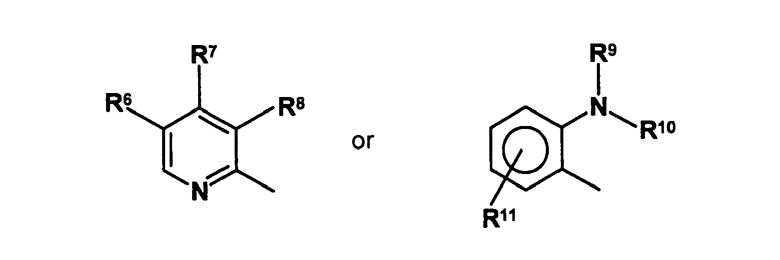 in which the residues R⁶, R⁷ and R⁸ are independently hydrogen, alkyl, alkylthio, alkoxy, halogen substituted alkoxy, alkoxyalkoxy, dialkylamino, piperidino, morpholino, halogen, phenylalkyl or phenylalkoxy, or one of said residues is joined with the residue R⁵ to give a condensed ring system, residues R⁹ and R¹⁰ are independently hydrogen, halogen or alkyl, and residue R¹¹ is hydrogen, halogen, trifluoromethyl, alkyl or alkoxy,
in which a prochiral sulfide of formula (II) 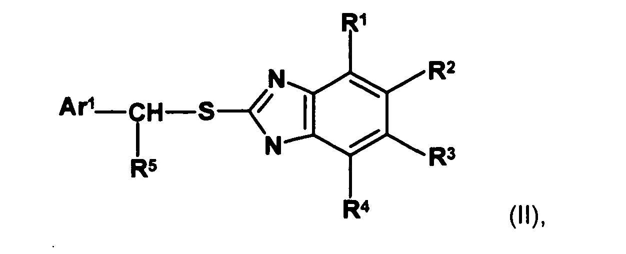 in which residues R¹, R², R³, R⁴, R⁵ and Ar¹ are as defined above, is oxidized in an organic solvent with an oxidant in the presence of a catalyst, **characterized in that** the catalyst is a titanium(IV) complex which can be obtained by reacting a titanium(IV) compound with a chiral, bidentate (R,R)- or (S,S)-1,2-bis-arylethane-1,2-diol at a temperature of from 20°C to 25°C over a period of time of 10 to 20 minutes, and wherein the catalytic reaction is carried out without adding a base.

2. The process according to claim 1, **characterized in that** the compound of formula (I) is a compound of formula 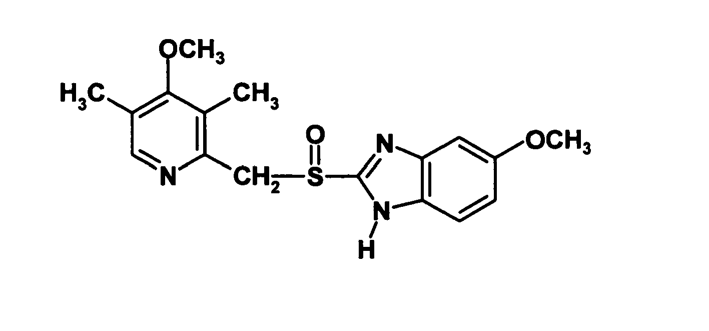 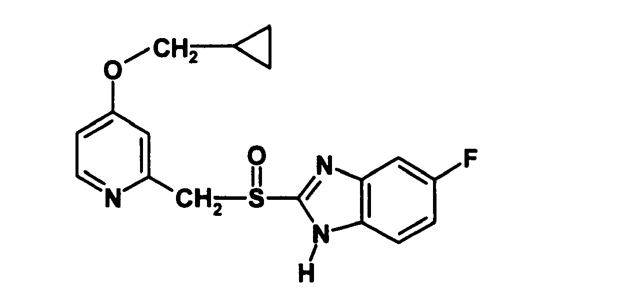 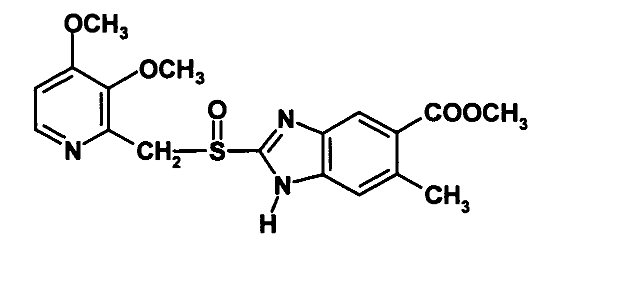 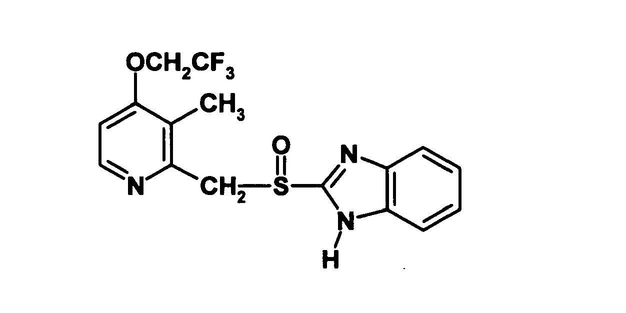 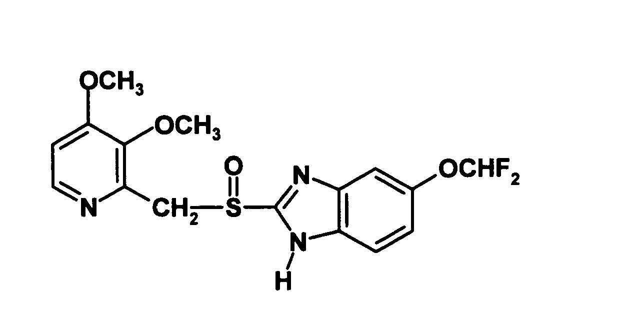 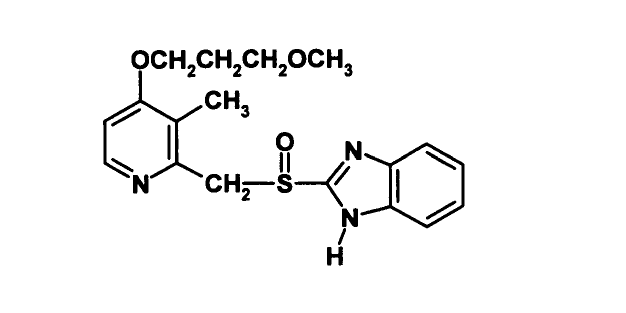 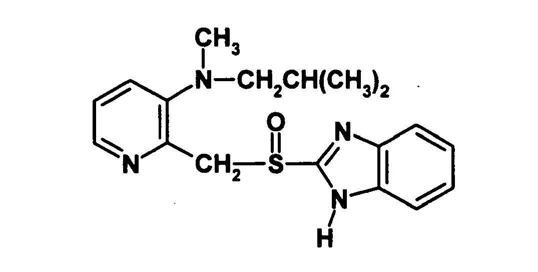 or 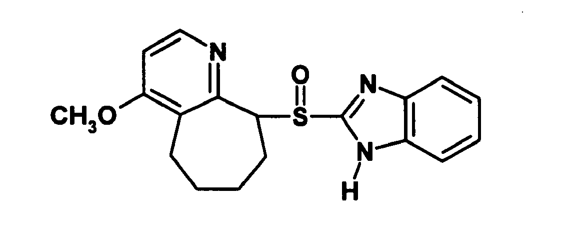 and the compound of formula (II) represents the corresponding prochiral sulfide.

3. The process according to claim 2 for the production of the S-enantiomer of omeprazole or a mixture of the S- and R-enantiomers of omeprazole in which the S-enantiomer of omeprazole is enriched.

4. The process according to claim 3, comprising the further step of reacting the S-enantiomer of omeprazole with a zinc source to give the zinc salt of the S-enantiomer of omeprazole.

5. The process according to any of claims 1 to 4, wherein the chiral, bidentate (R,R)- or (S,S)-1,2-bis-arylethane-1,2-diol is a compound of general formula (III) or (III') 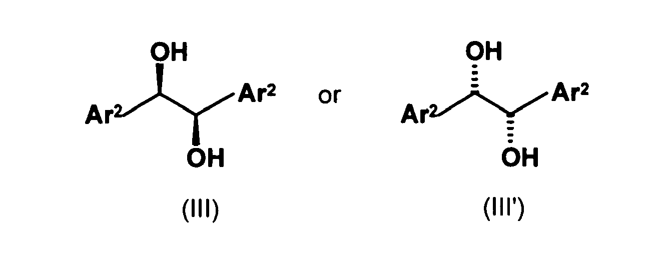 in which the residue Ar² is selected from 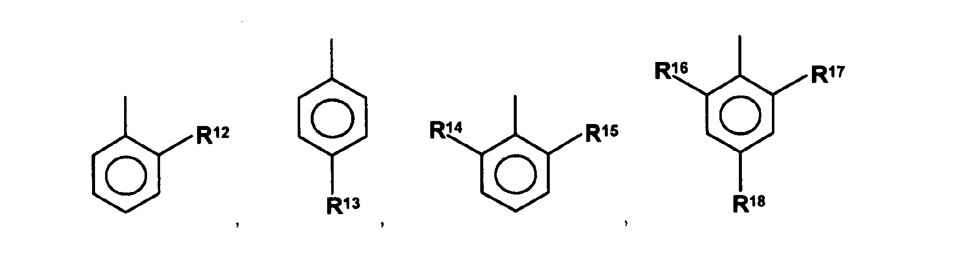 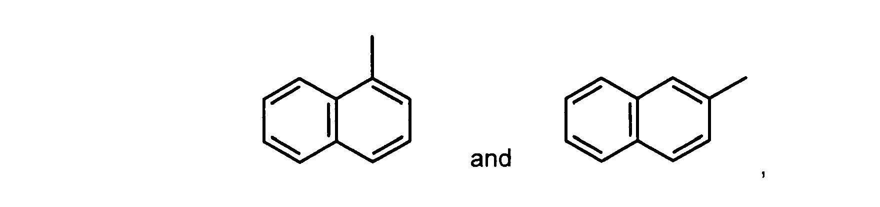 in which the residues R¹² to R¹⁸ are independently selected from hydrogen, alkyl, alkoxy, carboxylic acid ester residue, halogen, phenyl, trifluoromethyl and NO₂.

6. The process according to claim 5, wherein R¹⁴ and R¹⁵ are independently selected from hydrogen, alkyl, alkoxy and halogen, and the residues R¹⁶, R¹⁷ and R¹⁸ are independently selected from hydrogen and alkyl.

7. The process according to claim 6, wherein the residues R¹⁴ and R¹⁵ are equal and the residues R¹⁶, R¹⁷ and R¹⁸ are equal.

8. The process according to claim 7, wherein the residue R¹² is a bromine atom.

9. The process according to claim 8, wherein the (R,R)- or (S,S)-1,2-bis-aryl-1,2-diol is a compound of formula

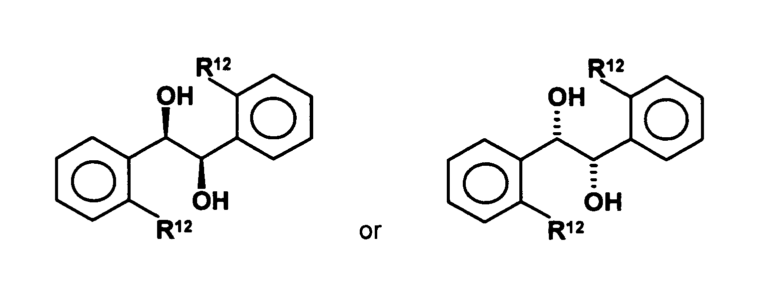

10. The process according to any of claims 1 to 9, wherein the titanium(IV) compound is an alkoxide of titanium(IV).

11. The process according to claim 10, wherein the titanium compound is the isopropoxide of titanium(IV).

12. The process according to any of the preceding claims, wherein the ratio of chiral, bidentate ligand to prochiral sulfide of formula (II) is within the range of 0.1:1.

13. The process according to any of the preceding claims, wherein the molar ratio of titanium(IV) alkoxide to prochiral sulfide of formula (II) is within the range of 0.05:1.

14. The process according to any of the preceding claims, wherein the reaction is carried out in the presence of water.

15. The process according to any of the preceding claims, wherein the oxidant is hydrogen peroxide, an alkyl hydroperoxide or an arylalkyl hydroperoxide.

16. The process according to any of the preceding claims, wherein the catalyst is produced by reacting the chiral ligand with the titanium(IV) alkoxide in an organic solvent before the prochiral sulfide of formula (II) is added to the reaction mixture.

17. The process according to any of the preceding claims, wherein the oxidation is carried out at about -20°C over a period of 12 to 18 hours.

18. The process according to any of the preceding claims, comprising the following steps:
a) providing a mixture of the chiral, bidentate (R,R)- or (S,S)-1,2-bis-arylethane-1,2-diol with the titanium(IV) alkoxide in the presence of an organic solvent,
b) adding water to the mixture of step a),
c) adding the prochiral sulfide of formula (II) to the reaction mixture of step b),
d) adding the oxidant to the reaction mixture of step c),
e) adding aqueous ammonia to the reaction mixture of step d),
f) adding an acid to the aqueous mixture of step e),
g) extracting the aqueous mixture using an organic solvent,
h) cooling the organic solution and isolating the precipitated enantiomer of the compound of formula (I) by filtration, and
i) where appropriate, converting the desired isomer of the compound of formula (I) into the zinc salt.

## Revendications

1. Procédé de production d'un énantiomère optiquement actif ou d'une forme enrichie en énantiomères d'un composé de formule (I) suivante : 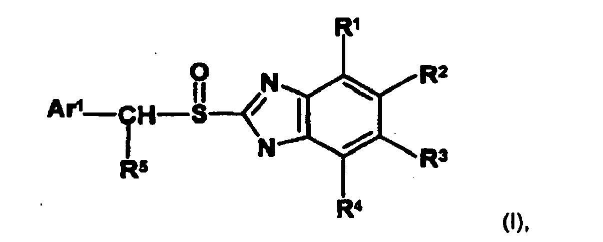 dans laquelle les radicaux R¹, R², R³ et R⁴ représentent,
indépendamment l'un de l'autre, l'hydrogène, un groupement alkyle, alcoxy, un halogène, un groupement halogénoalcoxy, alkylcarbonyle, alcoxycarbonyle, oxazolyle ou halogénoalkyle ou les radicaux adjacents R¹, R², R³ et R⁴ forment des structures cycliques éventuellement substituées, R⁵ représente un atome d'hydrogène ou est lié au radical Ar¹ pour former un système cyclique condensé et Ar¹ représente un radical de formule suivante : 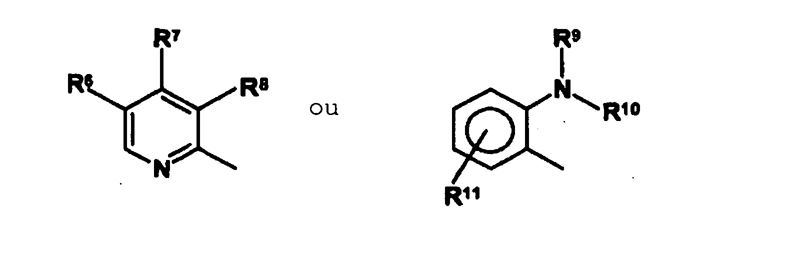 dans laquelle les radicaux R⁶, R⁷ et R⁸ représentent,
indépendamment l'un de l'autre, l'hydrogène, un groupement alkyle, alkylthio, alcoxy, alcoxy substitué par un halogène, alcoxyalcoxy, dialkylamino, pipéridino, morpholino, un halogène, un groupement phénylalkyle ou phénylalcoxy ou l'un de ces radicaux est lié au radical R⁵ pour former un système cyclique condensé, les radicaux R⁹ et R¹⁰ représentent, indépendamment l'un de l'autre, l'hydrogène, un halogène ou un alkyle et le radical R¹¹ représente l'hydrogène, un halogène, un groupement trifluorométhyle, alkyle ou alcoxy, dans lequel un sulfure prochiral de formule (II) suivante : 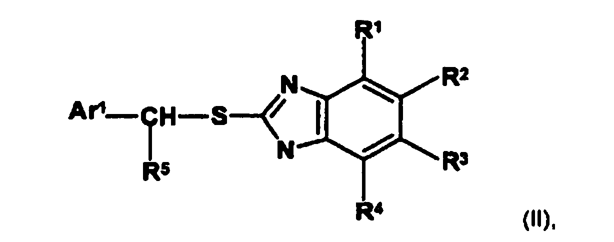 dans laquelle les radicaux R¹, R², R³, R⁴, R⁵ et Ar¹ sont définis comme précédemment, est oxydé dans un solvant organique avec un agent oxydant, en présence d'un catalyseur, **caractérisé en ce que** le catalyseur est un complexe de titane (IV), qui peut être obtenu en faisant réagir un composé de titane (IV) avec un (R,R)- ou (S,S)-1,2-bis-aryléthan-1,2-diol chiral bidentate à une température dans la plage de 20 °C à 25 °C sur une période de temps de 10 à 20 minutes, et dans lequel l'oxydation catalytique est réalisée sans ajouter de base.

2. Procédé selon la revendication 1, **caractérisé en ce que** le composé de formule (I) est un composé de formule suivante : 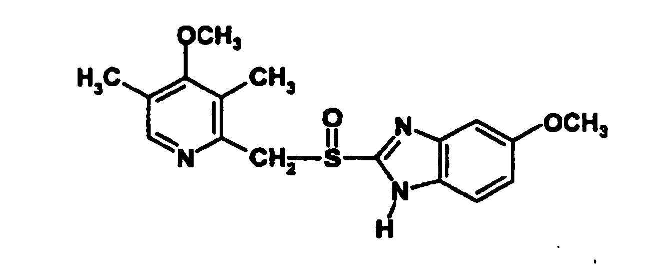 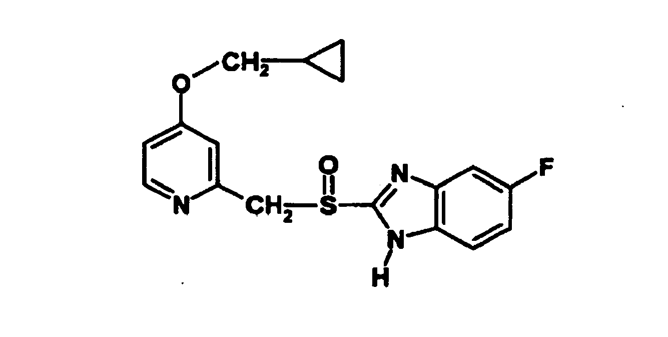 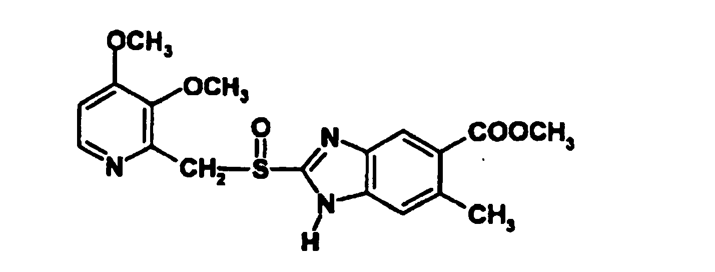 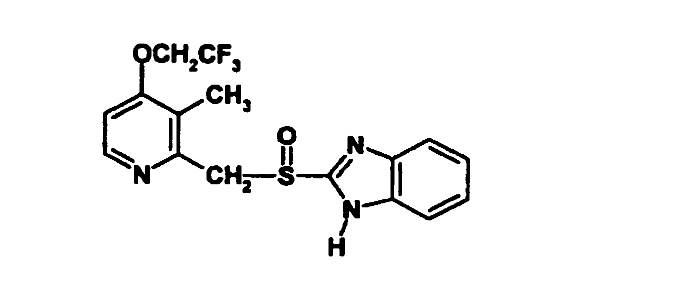 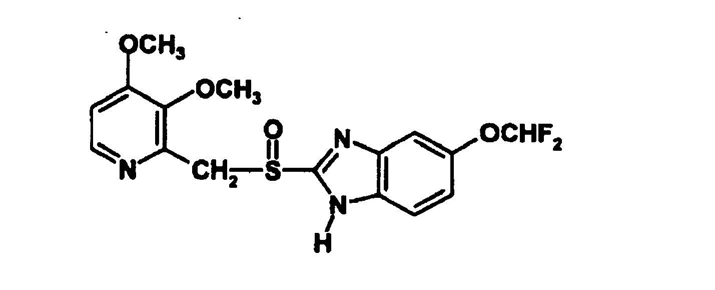 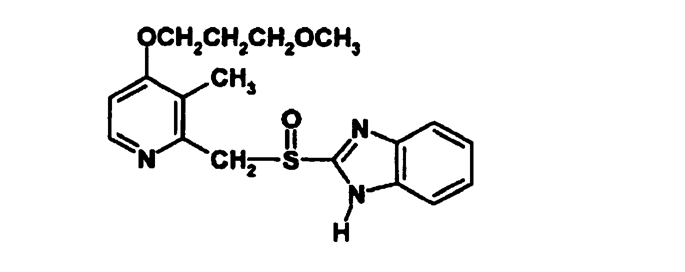 ou 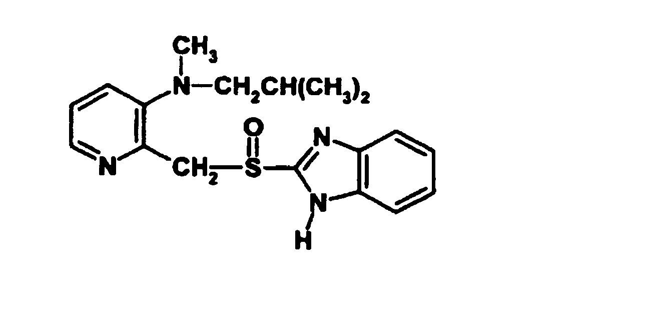 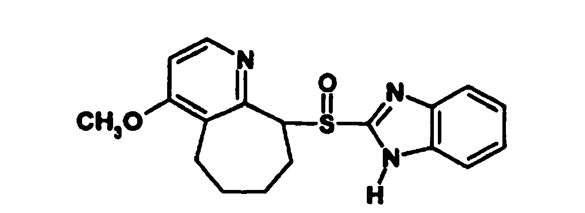 et **en ce que** le composé de formule (II) représente le sulfure prochiral correspondant.

3. Procédé selon la revendication 2 pour la fabrication de l'énantiomère S de l'oméprazole ou d'un mélange constitué de l'énantiomère S et de l'énantiomère R de l'oméprazole, dans lequel l'énantiomère S de l'oméprazole est enrichi.

4. Procédé selon la revendication 3, qui comprend l'étape suivante consistant à faire réagir l'énantiomère S de l'oméprazole avec une source de zinc de manière à former le sel de zinc de l'énantiomère S de l'oméprazole.

5. Procédé selon l'une quelconque des revendications 1 à 4, dans lequel le (R,R)- ou (S,S)-1,2-bis-aryléthan-1,2-diol chiral bidentate est un composé de formule générale (III) ou (III') suivante : 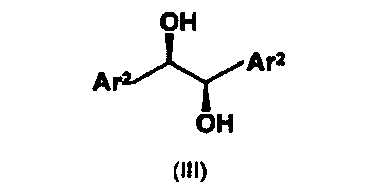 ou 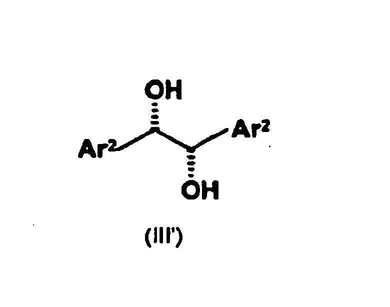 dans laquelle le radical Ar² est choisi parmi les composés
suivants : 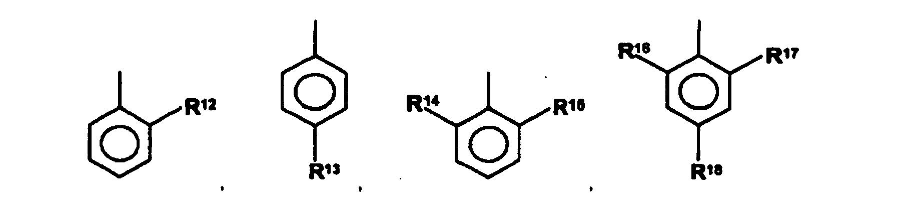 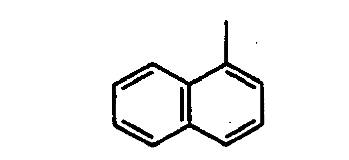 et 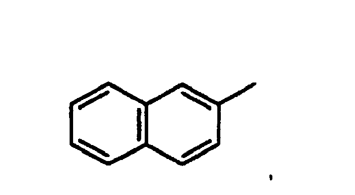 les radicaux R¹² à R¹⁸ étant choisis, indépendamment l'un de l'autre, parmi l'hydrogène, un groupement alkyle, alcoxy, un radical ester d'acide carboxylique, un halogène, un groupement phényle, trifluorométhyle et NO₂.

6. Procédé selon la revendication 5, dans lequel les radicaux R¹⁴ et R¹⁵ sont choisis, indépendamment l'un de l'autre, parmi l'hydrogène, un groupement alkyle, alcoxy et un halogène et les radicaux R¹⁶, R¹⁴ et R¹⁸ sont choisis, indépendamment l'un de l'autre, parmi l'hydrogène et un alkyle.

7. Procédé selon la revendication 6, dans lequel les radicaux R¹⁴ et R¹⁵ sont identiques et les radicaux R¹⁶, R¹⁷ et R¹⁸ sont identiques.

8. Procédé selon la revendication 7, dans lequel le radical R¹² représente un atome de brome.

9. Procédé selon la revendication 8, dans lequel le (R, R) - ou (S,S)-1,2-bis-aryl-1,2-diol est un composé de formule suivante : 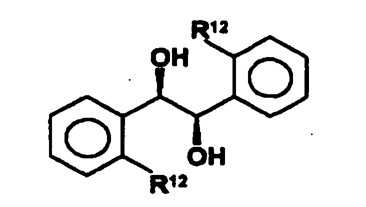 ou

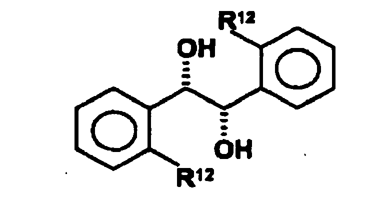

10. Procédé selon l'une quelconque des revendications 1 à 9, dans lequel le composé de titane (IV) est un alcoxyde de titane (IV).

11. Procédé selon la revendication 10, dans lequel le composé de titane est l' isopropoxyde de titane (IV).

12. Procédé selon l'une quelconque des revendications précédentes, dans lequel le rapport du ligand chiral bidentate au sulfure prochiral de formule (II) se situe dans la plage de 0,1:1.

13. Procédé selon l'une quelconque des revendications précédentes, dans lequel le rapport molaire de l'alcoxyde de titane (IV) au sulfure prochiral de formule (II) se situe dans la plage de 0,05:1.

14. Procédé selon l'une quelconque des revendications précédentes, dans lequel la réaction est réalisée en présence d'eau.

15. Procédé selon l'une quelconque des revendications précédentes, dans lequel l'agent oxydant est le
peroxyde d'hydrogène, un hydroperoxyde d'alkyle ou un hydroperoxyde d'arylalkyle.

16. Procédé selon l'une quelconque des revendications précédentes, dans lequel le catalyseur est produit en faisant réagir le ligand chiral avec l'alcoxyde de titane (IV) dans un solvant organique, avant d'ajouter le sulfure prochiral de formule (II) au mélange réactionnel.

17. Procédé selon l'une quelconque des revendications précédentes, dans lequel l'oxydation est réalisée à une température d'environ -20 °C sur une période de temps dans la plage de 12 à 18 heures.

18. Procédé selon l'une quelconque des revendications précédentes, comprenant les étapes suivantes consistant à :
a) incorporer un mélange du (R, R) - ou (S,S)-1,2-bis-aryléthan-1,2-diol chiral bidentate à l'alcoxyde de titane (IV) en présence d'un solvant organique,
b) ajouter de l'eau au mélange de l'étape a),
c) ajouter le sulfure prochiral de formule (II) au mélange réactionnel de l'étape b),
d) ajouter l'agent oxydant au mélange réactionnel de l'étape c),
e) ajouter une solution aqueuse d'ammoniac au mélange réactionnel de l'étape d),
f) ajouter un acide au mélange aqueux de l'étape e),
g) extraire le mélange aqueux avec un solvant organique,
h) refroidir le solvant organique et séparer par filtration l'énantiomère précipité du composé de formule (I), et
i) éventuellement, transformer l'isomère souhaité du composé de formule (I) en sel de zinc.
